# EUROPEAN PATENT SPECIFICATION

(11) **EP 4 376 938 B1**
(45) Date of publication and mention of the grant of the patent: **09.09.2026**
(21) Application number: 22789804.6
(22) Date of filing: 16.09.2022
(51) Int. Cl.: A61N 1/05, A61N 1/36, A61N 1/372, A61B 5/377, A61B 5/00, A61B 5/389

(54) **EVOKED POTENTIALS FOR BRAIN STIMULATION THERAPIES**
EVOZIERTE POTENZIALE FÜR HIRNSTIMULATIONSTHERAPIEN
POTENTIELS ÉVOQUÉS POUR THÉRAPIES DE STIMULATION CÉRÉBRALE

(30) Priority: 24.09.2021 US 202163261584 P
(43) Date of publication of application: 05.06.2024
(73) Proprietor: Boston Scientific Neuromodulation Corporation, Valencia, CA 91355 (US)
(72) Inventor: ESTELLER, Rosana, Santa Clarita, CA 91390 (US); MALEKMOHAMMADI, Mahsa, Sherman Oaks, CA 91411 (US); SERRANO CARMONA, Raul, Venice, CA 90291 (US)
(74) Representative: Vossius & Partner Patentanwälte Rechtsanwälte mbB
(86) International application number: PCT/US2022/076574
(87) International publication number: WO 2023/049669

(56) References cited:
- WO-A1-2016/205231
- WO-A1-2019/210371
- WO-A2-2018/008034
- US-A1- 2009 118 786

## Description

### FIELD OF THE INVENTION

This application relates to deep brain stimulation (DBS), and more particularly, to systems for using sensed neural responses for facilitating aspects of DBS.

### INTRODUCTION

Implantable neurostimulator devices are devices that generate and deliver electrical stimuli to body nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and deep brain stimulators to treat motor and psychological disorders, and other neural stimulators to treat urinary incontinence, sleep apnea, shoulder subluxation, etc. The description that follows will generally focus on the use of the invention within Deep Brain Stimulation (DBS). DBS has been applied therapeutically for the treatment of neurological disorders, including Parkinson's Disease, essential tremor, dystonia, and epilepsy, to name but a few. Further details discussing the treatment of diseases using DBS are disclosed in U.S. Pat. Nos. 6,845,267, 6,845,267, and 6,950,707. WO 2019/210371 A1 discloses systems and methods for monitoring neural activity. A method of monitoring neural activity responsive to a stimulus in a brain or a subject under general anaesthetic disclosed in WO 2019/210371 A1 comprises: applying the stimulus to one or more of at least one electrode implanted in a target neural structure of the brain; detecting a resonant response from the target neural structure evoked by the stimulus at one or more of the at least one electrode in or near the target neural structure of the brain; and determining one or more waveform characteristics of the detected resonant response. However, the present invention may find applicability with any implantable neurostimulator device system.

Each of these neurostimulation systems, whether implantable or external, typically includes one or more electrode carrying stimulation leads, which are implanted at the desired stimulation site, and a neurostimulator, used externally or implanted remotely from the stimulation site, but coupled either directly to the neurostimulation lead(s) or indirectly to the neurostimulation lead(s) via a lead extension. The neurostimulation system may further comprise a handheld external control device to remotely instruct the neurostimulator to generate electrical stimulation pulses in accordance with selected stimulation parameters. Typically, the stimulation parameters programmed into the neurostimulator can be adjusted by manipulating controls on the external control device to modify the electrical stimulation provided by the neurostimulator system to the patient.

Thus, in accordance with the stimulation parameters programmed by the external control device, electrical pulses can be delivered from the neurostimulator to the stimulation electrode(s) to stimulate or activate a volume of tissue in accordance with a set of stimulation parameters and provide the desired efficacious therapy to the patient. The best stimulus parameter set will typically be one that delivers stimulation energy to the volume of tissue or neural pathways that must be stimulated in order to provide the therapeutic benefit (e.g., treatment of movement disorders), while minimizing the volume of non-target tissue or neural pathways that are stimulated. A typical stimulation parameter set may include the electrodes that are acting as anodes or cathodes, as well as the amplitude, duration, and rate of the stimulation pulses.

Non-optimal electrode placement and stimulation parameter selections may result in excessive energy consumption due to stimulation that is set at too high an amplitude, too wide a pulse duration, or too fast a frequency; inadequate or marginalized treatment due to stimulation that is set at too low an amplitude, too narrow a pulse duration, or too slow a frequency; or stimulation of neighboring neural populations or other areas remote to the stimulation site via connecting neural pathways that may result in undesirable side effects. For example, bilateral DBS of the subthalamic nucleus (STN) has been shown to provide effective therapy for improving the major motor signs of advanced Parkinson's disease, and although the bilateral stimulation of the subthalamic nucleus is considered safe, an emerging concern is the potential negative consequences that it may have on cognitive functioning and overall quality of life (see A. M. M. Frankemolle, et al., Reversing Cognitive-Motor Impairments in Parkinson's Disease Patients Using a Computational Modelling Approach to Deep Brain Stimulation Programming, Brain 2010; pp. 1-16). In large part, this phenomenon is due to the small size of the STN. Even with the electrodes are located predominately within the sensorimotor territory, the electrical field generated by DBS is non-discriminately applied to all neural elements surrounding the electrodes, thereby resulting in the spread of current to neural elements affecting cognition. As a result, diminished cognitive function during stimulation of the STN may occur do to non-selective activation of non-motor pathways within or around the STN.

The large number of electrodes available, combined with the ability to generate a variety of complex stimulation pulses, presents a huge selection of stimulation parameter sets to the clinician or patient. In the context of DBS, neurostimulation leads with a complex arrangement of electrodes that not only are distributed axially along the leads, but are also distributed circumferentially around the neurostimulation leads as segmented electrodes, can be used.

To facilitate such selection, the clinician generally programs the external control device, and if applicable the neurostimulator, through a computerized programming system. This programming system can be a self-contained hardware/software system, or can be defined predominantly by software running on a standard personal computer (PC) or mobile platform. The PC or custom hardware may actively control the characteristics of the electrical stimulation generated by the neurostimulator to allow the optimum stimulation parameters to be determined based on patient feedback, including both, but not limited to, behavioral and clinical response, anatomical and neurophysiological information and to subsequently program the external control device with the optimum stimulation parameters.

When electrical leads are implanted within the patient, the computerized programming system may be used to instruct the neurostimulator to apply electrical stimulation to test placement of the leads and/or electrodes, thereby assuring that the leads and/or electrodes are implanted in effective locations within the patient. The system may also instruct the user how to improve the positioning of the leads, or confirm when a lead is well-positioned. Once the leads are correctly positioned, a fitting procedure, which may be referred to as a navigation session, may be performed using the computerized programming system to program the external control device, and if applicable the neurostimulator, with a set of stimulation parameters that best addresses the neurological disorder(s).

An aspect of programming the patient's stimulation parameters involves determining which electrodes to use to make electric fields that are best configured to treat the patient's symptoms and to avoid unwanted side effects. In the context of DBS, the leads are typically implanted into a particular region of the brain, such as the STN, as described below. Stimulation of that region may be effective at modulating the patient's symptoms. However, if intensity or amplitude of the stimulation becomes too great it may also stimulate nearby and/or remote non-target areas of the brain and cause side effects. Ideally, the clinician would like to determine a position within the target area of the patient's brain and determine an electrode configuration that provides a large range of stimulation intensities (i.e., a large therapeutic window) without stimulating non-target areas. Thus, there is a need for methods and systems that assist a clinician in doing so.

### SUMMARY

The invention is defined by the independent claim 1. Disclosed herein is a non-claimed method of optimizing a location on an electrode lead implanted in a patient's brain for providing electrical stimulation to the patient, wherein the electrode lead comprises a plurality electrodes, the method comprising: using one or more of the plurality of electrodes to sequentially provide electrical stimulation at different locations on the electrode lead, for each stimulation location: using one or more of the plurality of electrodes to record first signals, wherein the first signals are indicative of electric potentials evoked in the patient's brain by the stimulation, and recording second signals, wherein the second signals are indicative of motor activity evoked by the stimulation, and selecting an optimized location on the electrode lead for providing therapeutic electrical stimulation based on the first and second signals. According to some embodiments, the recorded first signals are indicative of evoked resonant neural responses evoked by the stimulation. According to some embodiments, the second signals are generated using electromyography (EMG), one or more mechanical sensors, speech sensors, and/or electrochemical sensors. According to some embodiments, the second signals are generated using a cortical array. According to some embodiments, the electric potentials evoked in the patient's brain are correlated with therapeutic efficacy of the stimulation. According to some embodiments, the motor activity evoked by the stimulation is correlated with an undesirable side effect of the stimulation. According to some embodiments, selecting an optimized location on the electrode lead based on the first and second signals comprises using the first and second signals to determine a therapeutic window for each of the stimulation locations. According to some embodiments, the first signals are indicative of potentials evoked in the patient's subthalamic nucleus (STN). According to some embodiments, the second signals are indicative of recruitment of neural elements in the patient's corticospinal tract by the stimulation. According to some embodiments, selecting an optimized location on the electrode lead based on the first and second signals comprises: for each stimulation location determining a value for a feature of the first signal and a value for a feature of the second signal, selecting a plurality of stimulation locations where the value for the feature of the second signals is less than a threshold value, and selecting a stimulation location from the plurality of stimulation locations where the value for the feature of the first signal is the greatest. According to some embodiments, selecting an optimized location on the electrode lead based on the first and second signals comprises: for each stimulation location determining a ratio comprising a value for a feature of the first signal and a value for a feature of the second signal and comparing the ratio to a threshold value.

Also disclosed herein is a system for providing stimulation to a patient's brain using an electrode lead that is implantable in the patient's brain and comprises a plurality of electrodes, the system comprising: control circuitry configured to: use one or more of the plurality of electrodes to sequentially provide electrical stimulation at different locations on the electrode lead, for each stimulation location: use one or more of the plurality of electrodes to record first signals, wherein the first signals are indicative of electric potentials evoked in the patient's brain by the stimulation, and receive one or more second signals that are indicative of motor activity evoked by the stimulation, and select an optimized location on the electrode lead for providing therapeutic electrical stimulation based on the first and second signals. According to some embodiments, the recorded first signals are indicative of evoked resonant neural responses evoked by the stimulation. According to some embodiments, the second signals are generated using electromyography (EMG), one or more mechanical sensors, speech sensors, and/or electrochemical sensors. According to some embodiments, the second signals are generated using a cortical array. According to some embodiments, the electric potentials evoked in the patient's brain are correlated with therapeutic efficacy of the stimulation. According to some embodiments, the motor activity evoked by the stimulation is correlated with an undesirable side effect of the stimulation. According to some embodiments, selecting an optimized location on the electrode lead for based on the first and second signals comprises using the first and second signals to determine a therapeutic window for each of the stimulation locations. According to some embodiments, the first signals are indicative of potentials evoked in the patient's subthalamic nucleus (STN). According to some embodiments, the second signals are indicative of recruitment of neural elements in the patient's corticospinal tract by the stimulation. According to some embodiments, selecting an optimized location on the electrode lead based on the first and second signals comprises: for each stimulation location determining a value for a feature of the first signal and a value for a feature of the second signal, selecting a plurality of stimulation locations where the value for the feature of the second signals is less than a threshold value, and selecting a stimulation location from the plurality of stimulation locations where the value for the feature of the first signal is the greatest. According to some embodiments, selecting an optimized location on the electrode lead based on the first and second signals comprises: for each stimulation location determining a ratio comprising a value for a feature of the first signal and a value for a feature of the second signal and comparing the ratio to a threshold value.

Also disclosed herein is a non-claimed method of implanting a stimulation lead in the brain of a patient, wherein the stimulation lead comprises a plurality of electrodes, the method comprising: positioning the lead at a first position in the patient's brain, using one or more of the electrodes to apply stimulation to the patient's brain, using one or more of the plurality of electrodes to record first signals, wherein the first signals are indicative of electric potentials evoked in the patient's brain by the stimulation, recording second signals, wherein the second signals are indicative of motor activity evoked by the stimulation, and using the first and second signals to determine one or more of (i) whether to move the lead to a new position or (ii) to adjust stimulation parameters based on the evoked responses. According to some embodiments, the recorded first signals are indicative of evoked resonant neural responses evoked by the stimulation. According to some embodiments, the second signals are generated using electromyography (EMG), one or more mechanical sensors, speech sensors, and/or electrochemical sensors. According to some embodiments, the second signals are generated using a cortical array. According to some embodiments, the electric potentials evoked in the patient's brain are correlated with therapeutic efficacy of the stimulation. According to some embodiments, the motor activity evoked by the stimulation is correlated with an undesirable side effect of the stimulation. According to some embodiments, selecting an optimized location on the electrode lead for based on the first and second signals comprises using the first and second signals to determine a therapeutic window for each of the stimulation locations. According to some embodiments, the first signals are indicative of potentials evoked in the patient's subthalamic nucleus (STN). According to some embodiments, the second signals are indicative of recruitment of neural elements in the patient's corticospinal tract by the stimulation.

Also disclosed herein is a non-claimed method of optimizing one or more stimulation parameters for providing electrical stimulation to a patient's brain using an electrode lead implanted in the patient's brain, wherein the electrode lead comprises a plurality of electrodes, the method comprising: using one or more of the plurality of electrodes to provide stimulation to the patient's brain, using one or more of the plurality of electrodes to record first signals, wherein the first signals are indicative of electric potentials evoked in the patient's brain by the stimulation, recording second signals, wherein the second signals are indicative of motor activity evoked by the stimulation, and adjusting one or more parameters of the stimulation based on the first and second signals. According to some embodiments, the one or more parameters are one or more of an electrode configuration, an amplitude, a pulse width, and a frequency.

The invention may also reside in the form of a programed external device (via its control circuitry) for carrying out the above methods, a programmed IPG or ETS (via its control circuitry) for carrying out the above methods, a system including a programmed external device and IPG or ETS for carrying out the above methods, or as a computer readable media for carrying out the above methods stored in an external device or IPG or ETS. The invention may also reside in one or more non-transitory computer-readable media comprising instructions, which when executed by a processor of a machine configure the machine to perform any of the above methods.

### BRIEF DESCRIPTION OF THE DRAWINGS

Figure 1A shows an Implantable Pulse Generator (IPG).
Figure 1B shows a percutaneous lead having split-ring electrodes.
Figures 2A and 2B show an example of stimulation pulses (waveforms) producible by the IPG or by an External Trial Stimulator (ETS).
Figure 3 shows an example of stimulation circuitry useable in the IPG or ETS.
Figure 4 shows an ETS environment useable to provide stimulation before implantation of an IPG.
Figure 5 shows various external devices capable of communicating with and programming stimulation in an IPG or ETS.
Figure 6 illustrates sensing circuitry useable in an IPG.
Figure 7 illustrates an embodiment of a user interface (UI) for programming stimulation.
Figure 8 illustrates examples of evoked potentials (EPs).
Figure 9 illustrates a workflow for using EPs to inform implantation of an electrode lead in a patient's brain.
Figure 10 illustrates a system for implanting an electrode lead in a patient's brain..
Figure 11 illustrates a workflow for using EPs to determine an optimal position on a lead for providing stimulation to a neural target and for identifying optimal stimulation parameters.
Figure 12 illustrates a display of EP amplitude determined as a function of location on an electrode lead.
Figure 13 illustrates a workflow for using therapeutic EPs (TEPs) and side effect EPs (e.g., MEPs) to determine stimulation locations that maximize therapeutic benefit while minimizing side effects.
Figure 14 illustrates a display of TEPs and MEPs corresponding to different stimulation locations on an electrode lead.
Figure 15 illustrates shows an embodiment of using recorded TEPs and MEPs to determine a therapeutic window for a given stimulation location.
Figure 16 illustrates a display of therapeutic windows determined for different stimulation locations.

### DETAILED DESCRIPTION

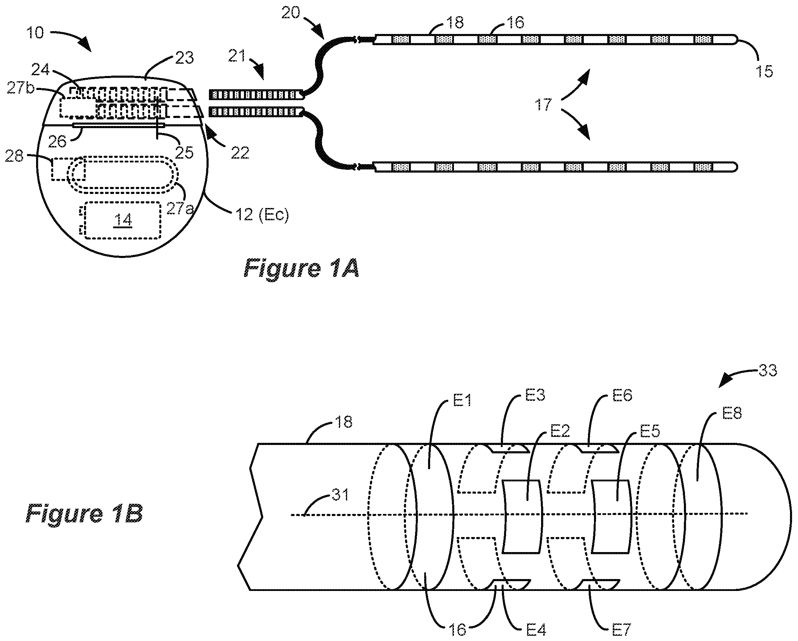

An implantable neurostimulator system, such as a DBS system, typically includes an Implantable Pulse Generator (IPG) 10 shown in Figure 1A. The IPG 10 includes a biocompatible device case 12 that holds the circuitry and a battery 14 for providing power for the IPG to function. The IPG 10 is coupled to tissue-stimulating electrodes 16 via one or more electrode leads that form an electrode array 17. For example, one or more electrode leads 15 can be used having ring-shaped electrodes 16 carried on a flexible body 18.

In yet another example shown in Figure 1B, an electrode lead 33 can include one or more split-ring electrodes. In this example, eight electrodes 16 (E1-E8) are shown, though the number of electrodes may vary. Electrode E8 at the distal end of the lead and electrode E1 at a proximal end of the lead comprise ring electrodes spanning 360 degrees around a central axis of the lead 33. Electrodes E2, E3, and E4 comprise split-ring electrodes, each of which are located at the same longitudinal position along the central axis 31, but with each spanning less than 360 degrees around the axis. For example, each of electrodes E2, E3, and E4 may span 90 degrees around the axis 31, with each being separated from the others by gaps of 30 degrees. Electrodes E5, E6, and E7 also comprise split-ring electrodes, but are located at a different longitudinal position along the central axis 31 than are split ring electrodes E2, E3, and E4. As shown, the split-ring electrodes E2-E4 and E5-E7 may be located at longitudinal positions along the axis 31 between ring electrodes E1 and E8. However, this is just one example of a lead 33 having split-ring electrodes. In other designs, all electrodes can be split-ring, or there could be different numbers of split-ring electrodes at each longitudinal position (i.e., more or less than three), or the ring and split-ring electrodes could occur at different or random longitudinal positions, etc.

Lead wires 20 within the leads are coupled to the electrodes 16 and to proximal contacts 21 insertable into lead connectors 22 fixed in a header 23 on the IPG 10, which header can comprise an epoxy for example. Once inserted, the proximal contacts 21 connect to header contacts 24 within the lead connectors 22, which are in turn coupled by feedthrough pins 25 through a case feedthrough 26 to stimulation circuitry 28 within the case 12, which stimulation circuitry 28 is described below.

In the IPG 10 illustrated in Figure 1A, there are sixteen electrodes (E1-E16), split between two percutaneous leads 15 (or contained on a single paddle lead, not shown) and thus the header 23 may include a 2x2 array of eight-electrode lead connectors 22. However, the type and number of leads, and the number of electrodes, in an IPG is application specific and therefore can vary. The conductive case 12 can also comprise an electrode (Ec).

In a DBS application, as is useful in the treatment of movement symptoms in Parkinson's disease for example, the IPG 10 is typically implanted under the patient's clavicle (collarbone). Lead wires 20 are tunneled through the neck and the scalp and the electrode leads 15 (or 33) are implanted through holes drilled in the skull and positioned for example in the subthalamic nucleus (STN) and the Globus pallidus intemus (GPi) in each brain hemisphere.

IPG 10 can include an antenna 27a allowing it to communicate bi-directionally with a number of external devices discussed subsequently. Antenna 27a as shown comprises a conductive coil within the case 12, although the coil antenna 27a can also appear in the header 23. When antenna 27a is configured as a coil, communication with external devices preferably occurs using near-field magnetic induction. IPG 10 may also include a Radio-Frequency (RF) antenna 27b. In Figure 1A, RF antenna 27b is shown within the header 23, but it may also be within the case 12. RF antenna 27b may comprise a patch, slot, or wire, and may operate as a monopole or dipole. RF antenna 27b preferably communicates using far-field electromagnetic waves, and may operate in accordance with any number of known RF communication standards, such as Bluetooth, Bluetooth Low Energy (BLE), as described in U.S. Patent Publication 2019/0209851, Zigbee, WiFi, MICS, and the like.

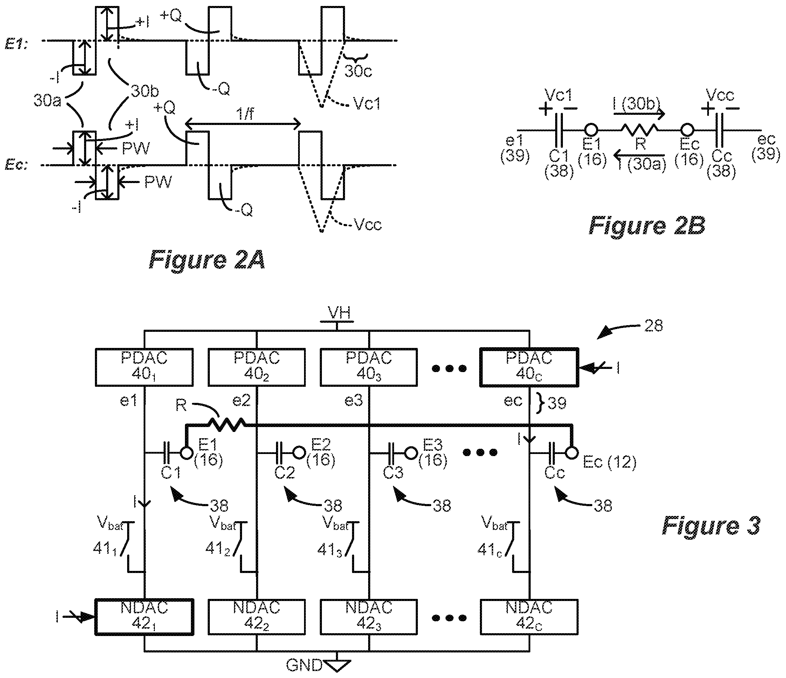

Stimulation in IPG 10 is typically provided by electrical pulses each of which may include a number of phases such as 30a and 30b, as shown in the example of Figure 2A. In the example shown, such stimulation is monopolar, meaning that a current is provided between at least one selected lead-based electrode (e.g., E1) and the case electrode Ec 12. Stimulation parameters typically include amplitude (current I, although a voltage amplitude V can also be used); frequency (f); pulse width (PW) of the pulses or of its individual phases such as 30a and 30b; the electrodes 16 selected to provide the stimulation; and the polarity of such selected electrodes, i.e., whether they act as anodes that source current to the tissue or cathodes that sink current from the tissue. These and possibly other stimulation parameters taken together comprise a stimulation program that the stimulation circuitry 28 in the IPG 10 can execute to provide therapeutic stimulation to a patient.

In the example of Figure 2A, electrode E1 has been selected as a cathode (during its first phase 30a), and thus provides pulses which sink a negative current of amplitude -I from the tissue. The case electrode Ec has been selected as an anode (again during first phase 30a), and thus provides pulses which source a corresponding positive current of amplitude +I to the tissue. Note that at any time the current sunk from the tissue (e.g., -I at E1 during phase 30a) equals the current sourced to the tissue (e.g., +I at Ec during phase 30a) to ensure that the net current injected into the tissue is zero. The polarity of the currents at these electrodes can be changed: Ec can be selected as a cathode, and E1 can be selected as an anode, etc.

IPG 10 as mentioned includes stimulation circuitry 28 to form prescribed stimulation at a patient's tissue. Figure 3 shows an example of stimulation circuitry 28, which includes one or more current sources 40ᵢ and one or more current sinks 42ᵢ. The sources and sinks 40ᵢ and 42ᵢ can comprise Digital-to-Analog converters (DACs), and may be referred to as PDACs 40ᵢ and NDACs 42ᵢ in accordance with the Positive (sourced, anodic) and Negative (sunk, cathodic) currents they respectively issue. In the example shown, a NDAC/PDAC 40ᵢ/42ᵢ pair is dedicated (hardwired) to a particular electrode node Ei 39. Each electrode node Ei 39 is connected to an electrode Ei 16 via a DC-blocking capacitor Ci 38, for the reasons explained below. PDACs 40ᵢ and NDACs 42ᵢ can also comprise voltage sources.

Proper control of the PDACs 40ᵢ and NDACs 42ᵢ allows any of the electrodes 16 and the case electrode Ec 12 to act as anodes or cathodes to create a current through a patient's tissue, R, hopefully with good therapeutic effect. In the example shown, and consistent with the first pulse phase 30a of Figure 2A, electrode E1 has been selected as a cathode electrode to sink current from the tissue R and case electrode Ec has been selected as an anode electrode to source current to the tissue R. Thus, PDAC 40c and NDAC 42₁ are activated and digitally programmed to produce the desired current, I, with the correct timing (e.g., in accordance with the prescribed frequency F and pulse width PW). Power for the stimulation circuitry 28 is provided by a compliance voltage VH, as described in further detail in U.S. Patent Application Publication 2013/0289665.

Other stimulation circuitries 28 can also be used in the IPG 10. In an example not shown, a switching matrix can intervene between the one or more PDACs 40ᵢ and the electrode nodes ei 39, and between the one or more NDACs 42ᵢ and the electrode nodes. Switching matrices allows one or more of the PDACs or one or more of the NDACs to be connected to one or more electrode nodes at a given time. Various examples of stimulation circuitries can be found in USPs 6,181,969, 8,606,362, 8,620,436, U.S. Patent Application Publications 2018/0071520 and 2019/0083796. The stimulation circuitries described herein provide multiple independent current control (MICC) (or multiple independent voltage control) to guide the estimate of current fractionalization among multiple electrodes and estimate a total amplitude that provides a desired strength. In other words, the total anodic current can be split among two or more electrodes and/or the total cathodic current can be split among two or more electrodes, allowing the stimulation location and resulting field shapes to be adjusted. For example, a "virtual electrode" may be created at a position between two physical electrodes by fractionating current between the two electrodes. In other words, the virtual electrode is not co-located with any of the physical electrodes. Appreciate, that in the context of split ring electrodes, such as electrodes E2-E4 (Fig. 1B), current fractionating can be used to create a virtual electrode at a rotational angle that is between two physical split ring electrodes (e.g., between E2 and E3). Accordingly, current fractionalization can be used to provide stimulation at any location along the lead and at any rotational angle about the lead. Note also that split ring electrodes at a given longitudinal position on the lead can be "ganged" together to effectively create a ring electrode at that position.

Much of the stimulation circuitry 28 of Figure 3, including the PDACs 40ᵢ and NDACs 42ᵢ, the switch matrices (if present), and the electrode nodes ei 39 can be integrated on one or more Application Specific Integrated Circuits (ASICs), as described in U.S. Patent Application Publications 2012/0095529, 2012/0092031, and 2012/0095519. As explained in these references, ASIC(s) may also contain other circuitry useful in the IPG 10, such as telemetry circuitry (for interfacing off chip with telemetry antennas 27a and/or 27b), circuitry for generating the compliance voltage VH, various measurement circuits, etc.

Also shown in Figure 3 are DC-blocking capacitors Ci 38 placed in series in the electrode current paths between each of the electrode nodes ei 39 and the electrodes Ei 16 (including the case electrode Ec 12). The DC-blocking capacitors 38 act as a safety measure to prevent DC current injection into the patient, as could occur for example if there is a circuit fault in the stimulation circuitry 28. The DC-blocking capacitors 38 are typically provided off-chip (off of the ASIC(s)), and instead may be provided in or on a circuit board in the IPG 10 used to integrate its various components, as explained in U.S. Patent Application Publication 2015/0157861.

Referring again to Figure 2A, the stimulation pulses as shown are biphasic, with each pulse comprising a first phase 30a followed thereafter by a second phase 30b of opposite polarity. Biphasic pulses are useful to actively recover any charge that might be stored on capacitive elements in the electrode current paths, such as on the DC-blocking capacitors 38. Charge recovery is shown with reference to both Figures 2A and 2B. During the first pulse phase 30a, charge will build up across the DC-blockings capacitors C1 and Cc associated with the electrodes E1 and Ec used to produce the current, giving rise to voltages Vc1 and Vcc which decrease in accordance with the amplitude of the current and the capacitance of the capacitors 38 (dV/dt = I/C). During the second pulse phase 30b, when the polarity of the current I is reversed at the selected electrodes E1 and Ec, the stored charge on capacitors C1 and Cc is actively recovered, and thus voltages Vc1 and Vcc increase and return to 0V at the end the second pulse phase 30b.

To recover all charge by the end of the second pulse phase 30b of each pulse (Vc1 = Vcc = 0V), the first and second phases 30a and 30b are charged balanced at each electrode, with the first pulse phase 30a providing a charge of -Q (-I * PW) and the second pulse phase 30b providing a charge of +Q (+I * PW) at electrode E1, and with the first pulse phase 30a providing a charge of +Q and the second pulse phase 30b providing a charge of -Q at the case electrode Ec. In the example shown, such charge balancing is achieved by using the same pulse width (PW) and the same amplitude (|I|) for each of the opposite-polarity pulse phases 30a and 30b. However, the pulse phases 30a and 30b may also be charged balance at each electrode if the product of the amplitude and pulse widths of the two phases 30a and 30b are equal, or if the area under each of the phases is equal, as is known.

Figure 3 shows that stimulation circuitry 28 can include passive recovery switches 41ᵢ, which are described further in U.S. Patent Application Publications 2018/0071527 and 2018/0140831. Passive recovery switches 41ᵢ may be attached to each of the electrode nodes ei 39, and are used to passively recover any charge remaining on the DC-blocking capacitors Ci 38 after issuance of the second pulse phase 30b-i.e., to recover charge without actively driving a current using the DAC circuitry. Passive charge recovery can be prudent, because non-idealities in the stimulation circuitry 28 may lead to pulse phases 30a and 30b that are not perfectly charge balanced.

Therefore, and as shown in Figure 2A, passive charge recovery typically occurs after the issuance of second pulse phases 30b, for example during at least a portion 30c of the quiet periods between the pulses, by closing passive recovery switches 41ᵢ. As shown in Figure 3, the other end of the switches 41ᵢ not coupled to the electrode nodes ei 39 are connected to a common reference voltage, which in this example comprises the voltage of the battery 14, Vbat, although another reference voltage could be used. As explained in the above-cited references, passive charge recovery tends to equilibrate the charge on the DC-blocking capacitors 38 by placing the capacitors in parallel between the reference voltage (Vbat) and the patient's tissue. Note that passive charge recovery is illustrated as small exponentially decaying curves during 30c in Figure 2A, which may be positive or negative depending on whether pulse phase 30a or 30b have a predominance of charge at a given electrode.

Passive charge recovery 30c may alleviate the need to use biphasic pulses for charge recovery, especially in the DBS context when the amplitudes of currents may be lower, and therefore charge recovery less of a concern. For example, and although not shown in Figure 2A, the pulses provided to the tissue may be monophasic, comprising only a first pulse phase 30a. This may be followed thereafter by passive charge recovery 30c to eliminate any charge build up that occurred during the singular pulses 30a.

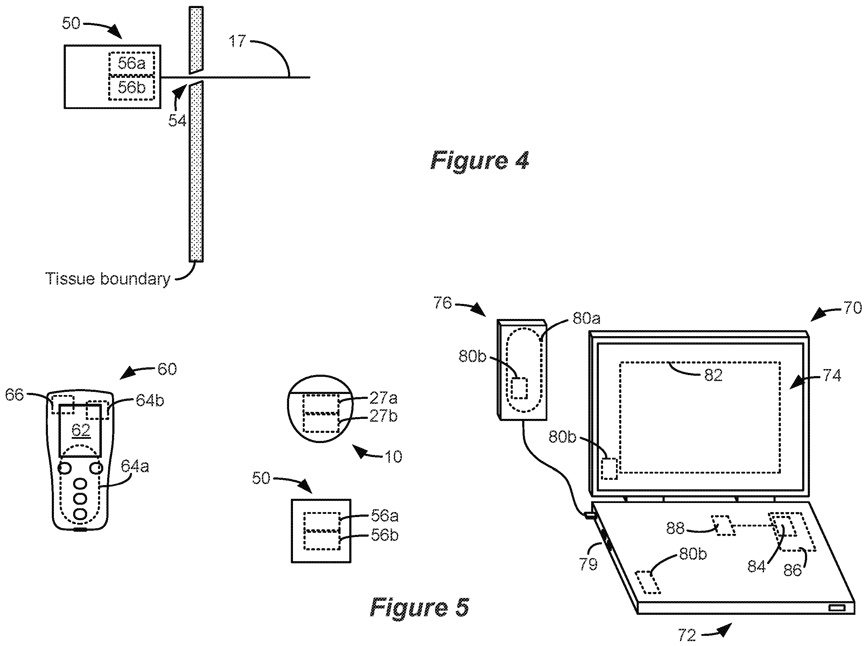

Figure 4 shows an external trial stimulation (ETS) that may be used prior to implantation of an IPG 10 in a patient, for example, in the operating room to test stimulation and confirm the lead position. During external trial stimulation, stimulation can be tried on the implant patient to evaluate therapeutic and side-effect thresholds and confirm that the lead is not too close to structures that cause side effects. Note that the term ETS, as used herein, refers broadly to any non-implanted device used to control the implanted leads to deliver stimulation, whether during the surgical implantation of the leads, during a fitting/programming session, etc. Like the IPG 10, the ETS 50 can include one or more antennas to enable bi-directional communications with external devices such as those shown in Figure 5. Such antennas can include a near-field magnetic-induction coil antenna 56a, and/or a far-field RF antenna 56b, as described earlier. ETS 50 may also include stimulation circuitry able to form stimulation in accordance with a stimulation program, which circuitry may be similar to or comprise the same stimulation circuitry 28 (Fig. 3) present in the IPG 10. ETS 50 may also include a battery (not shown) for operational power. The sensing capabilities described herein with regard to the IPG 10, may also be included in the ETS 50 for the purposes described below. As the IPG may include a case electrode, an ETS may provide one or more connections to establish similar returns; for example, using patch electrodes. Likewise, the ETS may communicate with the clinician programmer (CP) 70 so that the CP can process the data as described below.

Figure 5 shows various external devices that can wirelessly communicate data with the IPG 10 or ETS 50, including a patient hand-held external controller 60, and a clinician programmer (CP) 70. Both of devices 60 and 70 can be used to wirelessly transmit a stimulation program to the IPG 10 or ETS 50-that is, to program their stimulation circuitries to produce stimulation with a desired amplitude and timing described earlier. Both devices 60 and 70 may also be used to adjust one or more stimulation parameters of a stimulation program that the IPG 10 is currently executing. Devices 60 and 70 may also wirelessly receive information from the IPG 10 or ETS 50, such as various status information, etc.

External controller 60 can be as described in U.S. Patent Application Publication 2015/0080982 for example and may comprise a controller dedicated to work with the IPG 10 or ETS 50. External controller 60 may also comprise a general-purpose mobile electronics device such as a mobile phone, tablet, or other computing device that has been programmed with a Medical Device Application (MDA) allowing it to work as a wireless controller for the IPG 10 or ETS, as described in U.S. Patent Application Publication 2015/0231402. External controller 60 includes a user interface, preferably including means for entering commands (e.g., buttons or selectable graphical elements) and a display 62. The external controller 60's user interface enables a patient to adjust stimulation parameters, although it may have limited functionality when compared to the more-powerful clinician programmer 70, described shortly.

The external controller 60 can have one or more antennas capable of communicating with the IPG 10. For example, the external controller 60 can have a near-field magnetic-induction coil antenna 64a capable of wirelessly communicating with the coil antenna 27a or 56a in the IPG 10 or ETS 50. The external controller 60 can also have a far-field RF antenna 64b capable of wirelessly communicating with the RF antenna 27b or 56b in the IPG 10 or ETS 50.

Clinician programmer 70 is described further in U.S. Patent Application Publication 2015/0360038, and can comprise a computing device 72, such as a desktop, laptop, or notebook computer, a tablet, a mobile smart phone, a Personal Data Assistant (PDA)-type mobile computing device, etc. In Figure 5, computing device 72 is shown as a laptop computer that includes typical computer user interface means such as a screen 74, a mouse, a keyboard, speakers, a stylus, a printer, etc., not all of which are shown for convenience. Also shown in Figure 5 are accessory devices for the clinician programmer 70 that are usually specific to its operation as a stimulation controller, such as a communication "wand" 76 coupleable to suitable ports on the computing device 72, such as USB ports 79 for example.

The antenna used in the clinician programmer 70 to communicate with the IPG 10 or ETS 50 can depend on the type of antennas included in those devices. If the patient's IPG 10 or ETS 50 includes a coil antenna 27a or 56a, wand 76 can likewise include a coil antenna 80a to establish near-field magnetic-induction communications at small distances. In this instance, the wand 76 may be affixed in close proximity to the patient, such as by placing the wand 76 in a belt or holster wearable by the patient and proximate to the patient's IPG 10 or ETS 50. If the IPG 10 or ETS 50 includes an RF antenna 27b or 56b, the wand 76, the computing device 72, or both, can likewise include an RF antenna 80b to establish communication at larger distances. The clinician programmer 70 can also communicate with other devices and networks, such as the Internet, either wirelessly or via a wired link provided at an Ethernet or network port.

To program stimulation programs or parameters for the IPG 10 or ETS 50, the clinician interfaces with a clinician programmer graphical user interface (GUI) 82 provided on the display 74 of the computing device 72. As one skilled in the art understands, the GUI 82 can be rendered by execution of clinician programmer software 84 stored in the computing device 72, which software may be stored in the device's non-volatile memory 86. Execution of the clinician programmer software 84 in the computing device 72 can be facilitated by control circuitry 88 such as one or more microprocessors, microcomputers, FPGAs, DSPs, other digital logic structures, etc., which are capable of executing programs in a computing device, and which may comprise their own memories. For example, control circuitry 88 can comprise an i5 processor manufactured by Intel Corp, as described at https://www.intel.com/ content/ www/ us/ en/ products/ processors/ core/ i5-processors.html. Such control circuitry 88, in addition to executing the clinician programmer software 84 and rendering the GUI 82, can also enable communications via antennas 80a or 80b to communicate stimulation parameters chosen through the GUI 82 to the patient's IPG 10.

The user interface of the external controller 60 may provide similar functionality because the external controller 60 can include similar hardware and software programming as the clinician programmer. For example, the external controller 60 includes control circuitry 66 similar to the control circuitry 88 in the clinician programmer 70 and may similarly be programmed with external controller software stored in device memory.

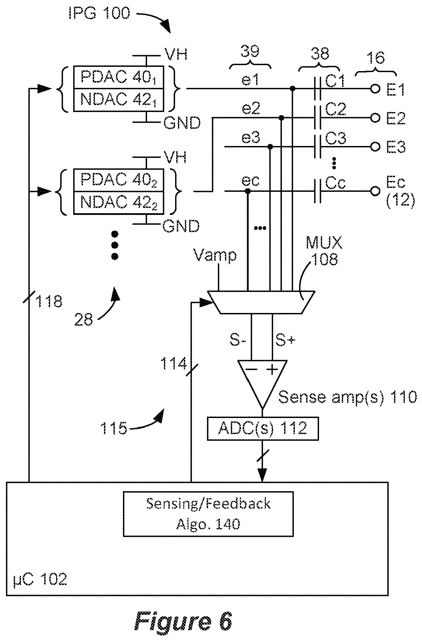

An increasingly interesting development in pulse generator systems is the addition of sensing capability to complement the stimulation that such systems provide. Figure 6 shows an IPG 100 that includes stimulation and sensing functionality. (An ETS as described earlier could also include stimulation and sensing capabilities). Figure 6 shows further details of the circuitry in an IPG 100 that can provide stimulation and sensing spontaneous or evoked signals. The IPG 100 includes control circuitry 102, which may comprise a microcontroller, such as Part Number MSP430, manufactured by Texas Instruments, Inc., which is described in data sheets at http:// www.ti.com/ microcontrollers/ msp430-ultra-low-power-mcus/ overview.html, which are incorporated herein by reference. Other types of controller circuitry may be used in lieu of a microcontroller as well, such as microprocessors, FPGAs, DSPs, or combinations of these, etc. Control circuitry 102 may also be formed in whole or in part in one or more Application Specific Integrated Circuits (ASICs), such as those described and incorporated earlier. The control circuitry 102 may be configured with one or more sensing/feedback algorithms 140 that are configured to cause the IPG to make certain adjustments and/or take certain actions based on the sensed neural signals.

The IPG 100 also includes stimulation circuitry 28 to produce stimulation at the electrodes 16, which may comprise the stimulation circuitry 28 shown earlier (Fig. 3). A bus 118 provides digital control signals from the control circuitry 102 to one or more PDACs 40ᵢ or NDACs 42ᵢ to produce currents or voltages of prescribed amplitudes (I) for the stimulation pulses, and with the correct timing (PW, F) at selected electrodes. As noted earlier, the DACs can be powered between a compliance voltage VH and ground. As also noted earlier, but not shown in Figure 4, switch matrices could intervene between the PDACs and the electrode nodes 39, and between the NDACs and the electrode nodes 39, to route their outputs to one or more of the electrodes, including the conductive case electrode 12 (Ec). Control signals for switch matrices, if present, may also be carried by bus 118. Notice that the current paths to the electrodes 16 include the DC-blocking capacitors 38 described earlier, which provide safety by preventing the inadvertent supply of DC current to an electrode and to a patient's tissue. Passive recovery switches 41ᵢ (Fig. 3) could also be present but are not shown in Figure 6 for simplicity.

IPG 100 also includes sensing circuitry 115, and one or more of the electrodes 16 can be used to sense spontaneous or evoked electrical signals, e.g., biopotentials from the patient's tissue. In this regard, each electrode node 39 can further be coupled to a sense amp circuit 110. Under control by bus 114, a multiplexer 108 can select one or more electrodes to operate as sensing electrodes (S+, S-) by coupling the electrode(s) to the sense amps circuit 110 at a given time, as explained further below. Although only one multiplexer 108 and sense amp circuit 110 are shown in Figure 6, there could be more than one. For example, there can be four multiplexer 108/sense amp circuit 110 pairs each operable within one of four timing channels supported by the IPG 100 to provide stimulation. The sensed signals output by the sense amp circuitry are preferably converted to digital signals by one or more Analog-to-Digital converters (ADC(s)) 112, which may sample the output of the sense amp circuit 110 at 50 kHz for example. The ADC(s) 112 may also reside within the control circuitry 102, particularly if the control circuitry 102 has A/D inputs. Multiplexer 108 can also provide a fixed reference voltage, Vamp, to the sense amp circuit 110, as is useful in a single-ended sensing mode (i.e., to set S- to Vamp).

So as not to bypass the safety provided by the DC-blocking capacitors 38, the inputs to the sense amp circuitry 110 are preferably taken from the electrode nodes 39. However, the DC-blocking capacitors 38 will pass AC signal components (while blocking DC components), and thus AC components within the signals being sensed will still readily be sensed by the sense amp circuitry 110. In other examples, signals may be sensed directly at the electrodes 16 without passage through intervening capacitors 38.

According to some embodiments, it may be preferred to sense signals differentially, and in this regard, the sense amp circuitry 110 comprises a differential amplifier receiving the sensed signal S+ (e.g., E3) at its non-inverting input and the sensing reference S- (e.g., E1) at its inverting input. As one skilled in the art understands, the differential amplifier will subtract S- from S+ at its output, and so will cancel out any common mode voltage from both inputs. This can be useful for example when sensing various neural signals, as it may be useful to subtract the relatively large-scale stimulation artifact from the measurement (as much as possible). Examples of sense amp circuitry 110, and manner in which such circuitry can be used, can be found in U.S. Patent Application Publication 2019/0299006; and U.S. Provisional Patent Application Serial Nos. 62/825,981, filed March 29, 2019; 62/825,982, filed March 29, 2019; and 62/883,452, filed August 6, 2019. The IPG (and/or ETS) may also be configured to determine impedances at any of the electrodes. For example, the IPG (and/or ETS) may be configured with sample and hold circuitry, controlled by the control circuitry for measuring impedances.

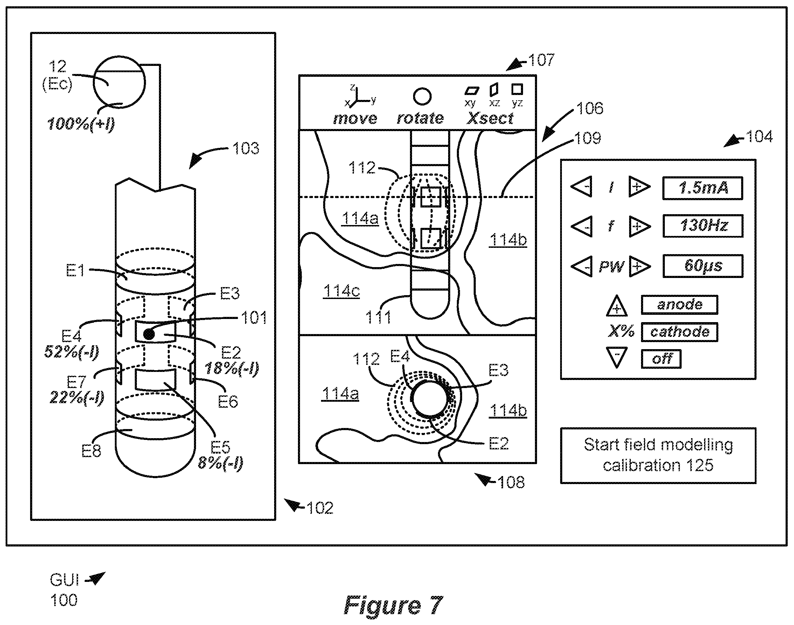

Particularly in the DBS context, it can be useful to provide a clinician with a visual indication of how stimulation selected for a patient will interact with the tissue in which the electrodes are implanted. This is illustrated in Figure 7, which shows a Graphical User Interface (GUI) 100 operable on an external device capable of communicating with an IPG 110 or ETS 150. Typically, and as assumed in the description that follows, GUI 100 would be rendered on a clinician programmer 70 (Fig. 5), which may be used during surgical implantation of the leads, or after implantation when a therapeutically useful stimulation program is being chosen for a patient. However, GUI 100 could be rendered on a patient external programmer 60 (Fig. 5) or any other external device capable of communicating with the IPG 110 or ETS 150.

GUI 100 allows a clinician (or patient) to select the stimulation program that the IPG 110 or ETS 150 will provide and provides options that control sensing of spontaneous or evoked responses, as described below. In this regard, the GUI 100 may include a stimulation parameter interface 104 where various aspects of the stimulation program can be selected or adjusted. For example, interface 104 allows a user to select the amplitude (e.g., a current I) for stimulation; the frequency (f) of stimulation pulses; and the pulse width (PW) of the stimulation pulses. Stimulation parameter interface104 can be significantly more complicated, particularly if the IPG 100 or ETS 150 supports the provision of stimulation that is more complicated than a repeating sequence of pulses. See, e.g., U.S. Patent Application Publication 2018/0071513. Nonetheless, interface 104 is simply shown for simplicity in Figure 7 as allowing only for amplitude, frequency, and pulse width adjustment. Stimulation parameter interface 104 may include inputs to allow a user to select whether stimulation will be provided using biphasic (Fig. 2A) or monophasic pulses, and to select whether passive charge recovery will be used, although again these details aren't shown for simplicity.

Stimulation parameter interface 104 may further allow a user to select the active electrodes-i.e., the electrodes that will receive the prescribed pulses. Selection of the active electrodes can occur in conjunction with a leads interface 102, which can include an image 103 of the one or more leads that have been implanted in the patient. Although not shown, the leads interface 102 can include a selection to access a library of relevant images 103 of the types of leads that may be implanted in different patients.

In the example shown in Figure 7, the leads interface 102 shows an image 103 of a single split-ring lead 33 like that described earlier with respect to Figure 1B. The leads interface 102 can include a cursor 101 that the user can move (e.g., using a mouse connected to the clinician programmer 70) to select an illustrated electrode 16 (e.g., E1-E8, or the case electrode Ec). Once an electrode has been selected, the stimulation parameter interface 104 can be used to designate the selected electrode as an anode that will source current to the tissue, or as a cathode that will sink current from the tissue. Further, the stimulation parameter interface 104 allows the amount of the total anodic or cathodic current +I or -I that each selected electrode will receive to be specified in terms of a percentage, X. For example, in Figure 7, the case electrode 12 Ec is specified to receive X=100% of the current I as an anodic current +I. The corresponding cathodic current -I is split between electrodes E2 (0.18*-I), E4 (0.52*-I), E5 (0.08*-I), and E7 (0.22*-I). Thus, two or more electrodes can be chosen to act as anodes or cathodes at a given time using MICC (as described above), allowing the electric field in the tissue to be shaped. The currents so specified at the selected electrodes can be those provided during a first pulse phase (if biphasic pulses are used), or during an only pulse phase (if monophasic pulses are used).

GUI 100 can further include a visualization interface 106 that can allow a user to view an indication of the effects of stimulation, such as electric field image 112 formed on the one or more leads given the selected stimulation parameters. The electric field image 112 is formed by field modelling in the clinician programmer 70. Only one lead is shown in the visualization interface 106 for simplicity, although again a given patient might be implanted with more than one lead. Visualization interface 106 provides an image 111 of the lead(s) which may be three-dimensional.

The visualization interface 106 preferably, but not necessarily, further includes tissue imaging information 114 taken from the patient, represented as three different tissue structures 114a, 114b and 114c in Figure 7 for the patient in question, which tissue structures may comprise different areas of the brain for example. Such tissue imaging information may comprise a Magnetic Resonance Image (MRI), a Computed Tomography (CT) image or other type of image and is preferably taken prior to implantation of the lead(s) in the patient. Often, one or more images, such as an MRI, CT, and/or a brain atlas are scaled and combined in a single image model. As one skilled in the art will understand, the location of the lead(s) can be precisely referenced to the tissue structures 114i because the lead(s) are implanted using a stereotactic frame (not shown). This allows the clinician programmer 70 on which GUI 100 is rendered to overlay the lead image 111 and the electric field image 112 with the tissue imaging information in the visualization interface 106 so that the position of the electric field 112 relative to the various tissue structures 114i can be visualized. The image of the patient's tissue may also be taken after implantation of the lead(s), or tissue imaging information may comprise a generic image pulled from a library which is not specific to the patient in question.

The various images shown in the visualization interface 106 (i.e., the lead image 111, the electric field image 112, and the tissue structures 114i) can be three-dimensional in nature, and hence may be rendered in the visualization interface 106 in a manner to allow such three-dimensionality to be better appreciated by the user, such as by shading or coloring the images, etc. Additionally, a view adjustment interface 107 may allow the user to move or rotate the images, using cursor 101 for example.

GUI 100 can further include a cross-section interface 108 to allow the various images to be seen in a two-dimensional cross section. Specifically, cross-section interface 108 shows a particular cross section 109 taken perpendicularly to the lead image 111 and through split-ring electrodes E2, E3, and E4. This cross section 109 can also be shown in the visualization interface 106, and the view adjustment interface 107 can include controls to allow the user to specify the plane of the cross section 109 (e.g., in XY, XZ, or YZ planes) and to move its location in the image. Once the location and orientation of the cross section 109 is defined, the cross-section interface 108 can show additional details. For example, the electric field image 112 can show equipotential lines allowing the user to get a sense of the strength and reach of the electric field at different locations. Although GUI 100 includes stimulation definition (102, 104) and imaging (108, 106) in a single screen of the GUI, these aspects can also be separated as part of the GUI 100 and made accessible through various menu selections, etc.

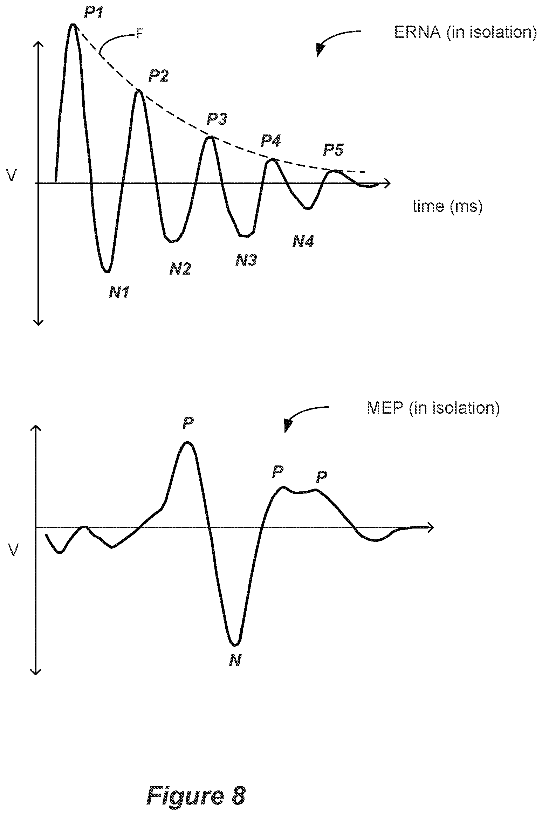

It has been observed that DBS stimulation in certain positions in the brain can evoke neural responses, i.e., electrical activity from neural elements, which may be measured either from brain itself or from other locations in the body (such as muscles). Such evoked neural responses are referred to herein generally as evoked potentials (EPs). One example of such EPs are resonant neural responses, referred to herein as evoked resonant neural responses (ERNAs). See, e.g., Sinclair, et al., "Subthalamic Nucleus Deep Brain Stimulation Evokes Resonant Neural Activity," Ann. Neurol. 83(5), 1027-31, 2018. The ERNA responses typically have an oscillation frequency of about 200 to about 500 Hz. Stimulation of the STN, and particularly of the dorsal subregion of the STN, has been observed to evoke strong ERNA responses, whereas stimulation of the posterior subthalamic area (PSA) does not evoke such responses. Thus, ERNA may provide a biomarker for electrode location, which can indicate acceptable or optimal lead placement and/or stimulation field placement for achieving the desired therapeutic response. An example of an ERNA in isolation is illustrated in Figure 8. The ERNA comprises a number of positive peaks Pₙ and negative peaks Nₙ, which may have characteristic amplitudes, separations, or latencies. The ERNA signal may decay according to a characteristic decay function F. Such characteristics of the ERNA response may provide indications of the brain activity associated with the neural response.

Another example of an evoked potentials are motor evoked potentials (MEPs), which are electrical signals recorded from the descending motor pathways or from muscles following stimulation of motor pathways in the brain. An MEP is shown in isolation in Figure 8, and comprises a number of peaks that are conventionally labeled with P for positive peaks and N for negative peaks. Note that not all MEPs will have the exact shape and number of peaks as illustrated in Figure 8.Other examples of electrical activity that may be recorded include spontaneous neural activity (local field potentials) as well as other evoked potentials, such as cortical evoked potentials, compound muscle action potentials (CMAPs), evoked compound action potentials (ECAPs), and the like.

Aspects of this disclosure relate to methods and systems for using evoked potentials, such as ERNA, MEPs and other evoked potentials, as well as other recorded electrical signals, such as local field potentials and/or spontaneous activity, to inform aspects of neuromodulation therapy, such as DBS therapy. The measurements described herein can be used during the surgical implantation of the electrode leads to help the clinician implant the lead in the desired location within the patient's brain. As used herein, the terms "therapeutic evoked potentials," "therapeutic EPs," or "TEPs" refer to EPs that are believed to be associated stimulation and/or lead placement that is likely to provide therapeutic benefit to the patient. As described in more detail below, the clinician may obtain measurements as a function of depth as they advance the lead from the entry point of the brain along the trajectory to the desired neural target to create a spatial profile of the evoked potentials along the trajectory. Once the clinician has determined that the lead is at an optimal location with respect to the target neural tissue, the methods and systems described herein can be used to determine the optimal stimulation location, with respect to both the longitudinal position along the lead and the angular position about the lead (using directional electrodes). MICC and current fractionalization can be used to provide center points of stimulation that are between physical electrodes.

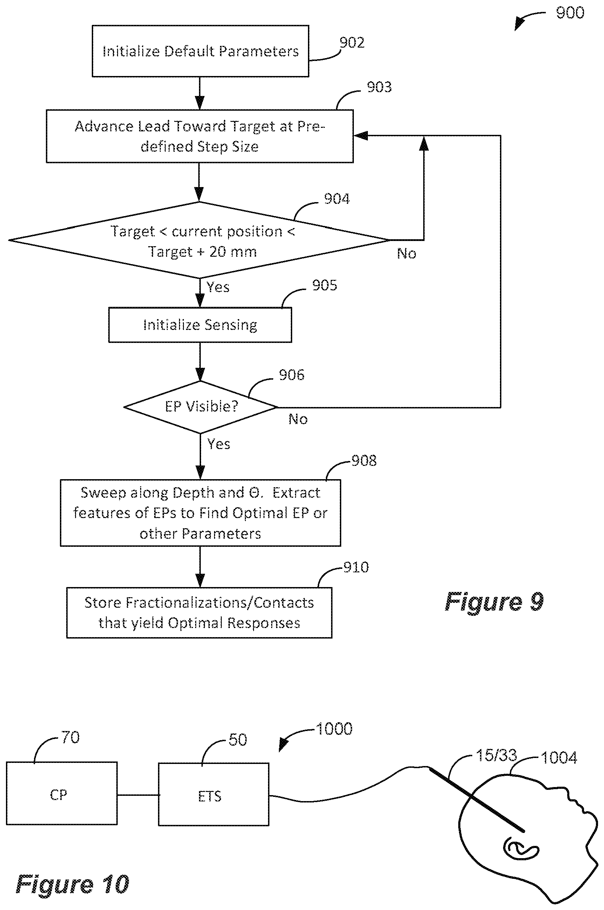

Figure 9 illustrates a workflow 900 that can be used to facilitate the surgical implantation of an electrode lead at a correct location in a patient's brain using EPs as a guide. At step 902, a set of default stimulation parameters can be initialized. The default stimulation parameters may correspond to stimulation parameters that are appropriate for therapeutic stimulation or the default parameters may be configured specifically for the evoked potential sensing workflow. For example, according to some embodiments, the default stimulation parameters optimized for sensing may comprise stimulation waveforms having active recharge (i.e., biphasic pulses) having short pulse widths (e.g., 50 µs or less) and comprising a long interphase interval (e.g., an interphase interval of 3 ms or greater). With such waveforms, the evoked potentials and other electrical measurements may be recorded during the interphase interval. Alternatively, the interphase interval could be shortened, and the recording can be conducted after the pulses. Still alternatively, monophasic pulses could be used. According to some embodiments, one or more bursts or envelopes of a plurality of pulse (e.g., ten pulses) may be used.

At step 903, the lead is advanced through the brain toward the target neural elements at a pre-defined step size. The step size may be on the order of 1 mm, for example. According to some embodiments, the target neural element(s) may comprise the STN, for example, the dorsolateral aspect of the STN, which is a common neural target in DBS. Other targets may include the patient's GPi. The lead may be advanced to within a certain pre-defined distance from the target neural elements. For example, the distance may be 20 mm from the target neural elements (step 904). Once the lead is within the pre-defined distance from the target neural elements sensing, as described above, may be initialized (step 905). Once sensing is initialized, it is determined whether evoked potentials can be detected (step 906). If evoked potentials are not detectable, the lead can be advanced further. Although not illustrated, a parameter sweep may be performed if evoked potentials are not detected. For example, the amplitude or other parameters of the stimulation may be adjusted in an attempt to elicit detectable EPs.

Once evoked potentials are detected, the stimulation may be swept along the longitudinal and angular (i.e., rotational) positions on the lead to determine the optimum stimulation location (step 908). For example, during the longitudinal sweep the electrode contacts at each longitudinal position can iteratively be used as the stimulating electrode and the other electrode contacts can be used as sensing/recording electrodes. According to some embodiments, directional electrodes at a given longitudinal location on the lead can be ganged together to act as a single ring electrode for stimulating and/or sensing during this step. MICC and current fractionalization can be used to provide stimulation at longitudinal locations between the electrodes. As each electrode contact from the proximal to the distal end of the lead is iteratively used as the stimulating electrode, evoked potentials are recorded at one or more of the other electrodes. This iterative process is used to create a comprehensive profile of the sensed evoked potentials relative to the locations upon the electrode lead both along and around the lead. One or more features of the evoked potentials can be extracted from the evoked potentials recorded at each of the sensing/recording electrodes. Generally, any value or metric may be used as the extracted feature(s). Examples of such features of the evoked potentials include but are not limited to:
- a height of any peak (e.g., N1);
- a peak-to-peak height between any two peaks (such as from N1 to P2);
- a ratio of peak heights (e.g., N1 / P2);
- a peak width of any peak (e.g., the full-width half-maximum of N1);
- an area or energy under any peak;
- a total area or energy comprising the area or energy under positive peaks with the area or energy under negative peaks subtracted or added;
- a length of any portion of the curve of the evoked potential (e.g., the length of the curve from P1 to N2);
- any time defining the duration of at least a portion of the evoked potential (e.g., the time from P1 to N2);
- latencies of any peaks (P1...Pn, N1...Nn, etc.) as well as other feature-to-feature latencies;
- amplitude decay function;
- a time delay from stimulation to issuance of the evoked potential, which is indicative of the neural conduction speed of the evoked potential, which can be different in different types of neural tissues;
- a conduction speed (i.e., conduction velocity) of the evoked potential, which can be determined by sensing the evoked potential as it moves past different sensing electrodes;
- a rate of variation of any of the previous features, i.e., how such features change over time;
- a power (or energy) determined in a specified frequency band (e.g., delta, alpha, beta, gamma, etc.) determined in a specified time window (for example, a time window that overlaps the neural response, the stimulation artifact, etc.);
- spectral characteristics in the frequency domain (e.g., Fourier transform);
- a cross-correlation or cross-coherence of the evoked potential shape with a target optimal shape; and
- any mathematical combination or function of these features.

Values for the one or more extracted features of the evoked potentials are determined as a function of the longitudinal stimulation locations along the electrode lead. The longitudinal location that yields the optimum value(s) for the one or more features can be selected as the best longitudinal location for providing stimulation. According to some embodiments, the optimum stimulation location may be the stimulation location that provides the maximum value of the evoked potential feature (such as evoked potential amplitude, peak height, etc.), indicating that stimulation at that location best activates targeted neural elements. The extracted features of the evoked potentials can be used to determine whether to advance the lead further with respect to the neural targets to obtain an optimum lead position. The decision to advance/reposition the lead may also be informed by measurements of EPs associated with side effects, such as motor EPs (MEPs), as described in more detail below. This is based on the consideration that the maximum TEP parameter may not necessarily indicate the best lead position because that lead position might be too close to neural structures that can cause side effects. So, as described in more detail below, embodiments described in this disclosure may seek to maximize TEP responses while minimizing EP responses associated with side effects. In other words, the disclosed methods and systems balance TEP response and MEP responses. At step 910, the stimulation locations, i.e., the fractionalizations determined for the longitudinal and rotational stimulation locations, can be saved and stored for later use.

Figure 10 illustrates a schematic of a system 1000 for performing implantation of an electrode lead (e.g., lead 15 or lead 33, Figs. 1A/1B) in the brain of a patient 1004, as described above (Fig. 9). The system 1000 also comprises one or more devices for controlling the stimulation and sensing provided at the electrode lead. The illustrated embodiment comprises a clinician programmer (CP) 70 for programming the stimulation and sensing parameters. The functionality of a CP 70 may be like that described above (FIG. 5), for example. The CP used during lead implantation may be the same machine or a different machine as the one used to program the patient's IPG later, during the fitting procedure. The clinician can use the CP 70 to select the electrodes of the lead 15/33 that will be used to provide stimulation, the parameters of the stimulation waveform(s) that will be applied, and the electrode(s) that will be used to sense evoked responses. In the illustrated system 1000, the CP 70 provides those selections to an ETS 50. The ETS 50 causes the stimulation to be applied to at leads. The ETS 50 also receives, and records sensed signals from the lead. The CP and ETS may communicate via a wired or a wireless connection. In the illustrated embodiment, a single ETS component is shown. However, according to some embodiments, multiple components could be used, for example, separate components for providing stimulation and for receiving and recording sensed signals. The CP may communicate with either or both ETS components in such an embodiment. According to some embodiments, aspects of the CP functionality and the ETS functionality may be combined in a single device. For example, the ETS 50 may itself be configured for programming the stimulation and/or sensing parameters. Alternatively, the functionality of receiving and recording the sensed signals (correlated with the stimulation configuration/parameters) may be embodied in the CP 70, for example as a module or subroutine additional to the CP functionality described above. Regardless of the exact configuration, the system is capable of causing stimulation of a defined waveform to be applied using selected one or more electrode on the lead, and of sensing/recording responses evoked by the stimulation. Further, the system 1000 (e.g., in either the CP 70 and/or the ETS 50) comprises control circuitry configured to perform the steps of the various algorithms and methods described with respect to Figure 9 and those described below. The control circuitry may be so configured by executing program code stored on non-volatile computer-readable media.

After the lead position is finalized, the clinician will seek to determine an electrode configuration that produces a best stimulation field for treating the patient's symptoms. As used herein the terms "electrode configuration," "configuration of electrode contacts," and the like are used to refer to how anodic and cathodic current is fractionalized among the electrode contacts to provide a particular stimulation field and/or stimulation at a particular center point of stimulation (CPS). In other words, the electrode configuration configuration/electrode contact configuration characterizes which electrodes/contacts are active, what is the polarity of each active electrode, and what is the relative strength of each active electrode.

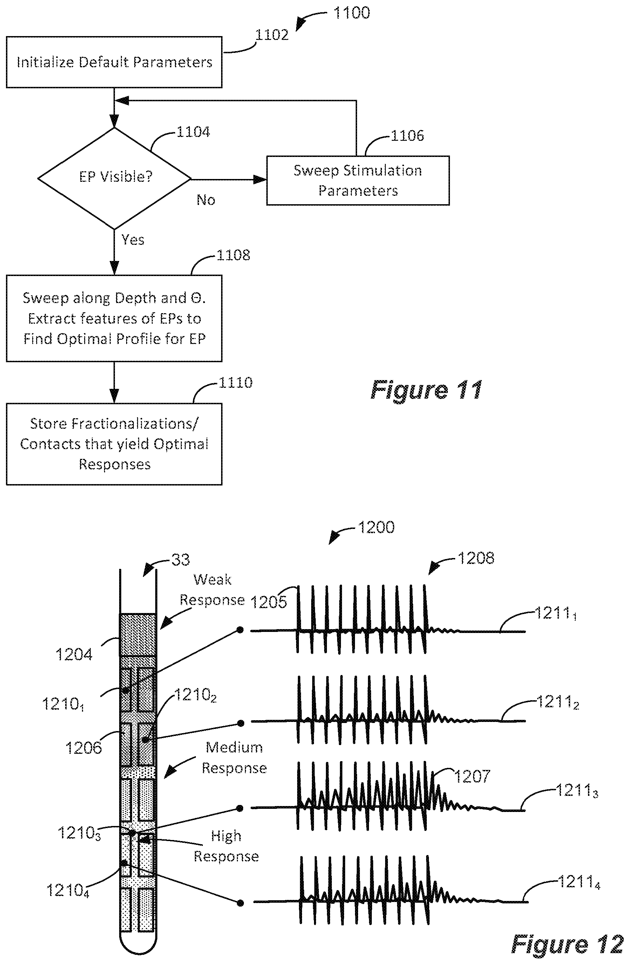

Figure 11 illustrates an example of a workflow 1100 for determining optimal contacts and/or current fractionalizations for providing stimulation to a patient. At step 1102 a set of default parameters may be initialized. The default parameters may be similar to the ones discussed above with respect to the workflow 900 (e.g., step 902, Fig. 9). According to some parameters, the default parameters may be parameters that were stored during the implantation (e.g., step 910, Fig. 9). At step 1104, one or more of the electrodes on the lead are used as sensing/recording electrodes to check for the presence of evoked potentials. According to some embodiments, all of the electrodes that are not being used to provide the stimulation are used as sensing/recording electrodes. According to some embodiments, electrodes on different leads from the stimulating electrode may be used for recording. Also note that spontaneous activity may be recorded without the need to stimulate. According to some embodiments, directional electrodes at a given longitudinal position on the lead are ganged together to function as a ring electrode for stimulation and/or sensing. If evoked potentials are not detected at one or more of the sensing/recording electrodes, the stimulation parameters may be modified (i.e., swept) to provide stimulation that evokes detectable response potentials (step 1106). For example, the amplitude of the stimulation waveform may be increased.

Once it is determined that the stimulation is providing usable evoked potentials, the stimulation may be swept along the longitudinal and angular (i.e., rotational) positions on the lead to determine the optimum stimulation location/electrode configuration (step 1108). For example, for the longitudinal sweep the electrode contacts at each longitudinal position can iteratively be used as the stimulating electrode and the other electrode contacts can be used as sensing/recording electrodes. Again, directional electrodes at a given longitudinal location on the lead can be ganged together to act as a single ring electrode for stimulating and/or sensing during this step. MICC and current fractionalization can be used to provide stimulation at longitudinal locations between the electrodes. As each electrode contact from the proximal to the distal end of the lead is iteratively used as the stimulating electrode, evoked potentials are recorded at one or more of the other electrodes. This iterative process is used to create a comprehensive profile of the sensed evoked potentials relative to the locations upon the electrode lead both along and around the lead. One or more features of the evoked potentials can be extracted from the evoked potentials recorded at each of the sensing/recording electrodes. Features of the evoked potentials can be extracted from the recorded signals, as described above. Values for the one or more extracted features of the evoked potentials are determined as a function of the longitudinal stimulation locations along the electrode lead. The longitudinal location that yields the optimum value(s) for the one or more features can be selected as the best longitudinal location for providing stimulation.

Once the optimum longitudinal stimulation location is determined, the sweeping process may be repeated to optimize the rotational stimulating location by iteratively using different directional electrodes (and/or fractionalized angular locations) to provide stimulation and using the other electrodes as sensing/recording electrodes to record evoked potentials. Again, one or more features may be extracted from the evoked potentials and the rotational position that yields the optimal values for the evoked potential features may be selected as the rotational location for providing directional stimulation. As described above, MICC and current fractionalization may be used to determine optimum stimulation locations that are located between physical locations of actual electrode contacts. Again, the rotational optimization may be performed using optimization algorithms or may be performed manually. At step 1110, the stimulation locations, i.e., the fractionalizations determined for the longitudinal and rotational stimulation locations, can be saved and stored.

Figure 12 illustrates an implanted lead 33 during an optimization process, as described above. In the illustrated example, the lead comprises 16 electrodes, including a single ring electrode 1204 and 15 segmented electrodes 1206. Examples of such leads and other suitable leads are described in U.S. Patent No. 10,286,205, the contents of which are incorporated herein by reference. Four stimulation locations, 1210₁₋₄ and the evoked signals recorded 1211₁₋₄ for each of those respective stimulation locations are illustrated. The recorded signals may comprise a stimulation artifact component 1205 and an EP component 1207. In the illustrated representation, the EP component is an oscillatory neural response, such as an ERNA response described above. It should be appreciated that the stimulation locations on the lead may or may not correspond to positions of physical electrodes. For example, MICC can be used, as described above, to provide stimulation at locations that do not directly coincide with physical electrodes, such as location 1210₃. In the illustration, stimulation at the location 1210₃ evokes the largest EP response. The availability of MICC to provide stimulation at any location on the lead provides high resolution for locating a stimulation location with a maximum ERNA response. The illustration also shows a "heat map" indicating the stimulation locations on the lead the evoke the highest and lowest EP responses.

Notice that the algorithm 1100 detailed in Figure 11 and illustrated in Figure 12 is agnostic to the presence of side effects even though side effects can be problematic with many DBS modalities. For example, as mentioned above, a target for DBS therapy may be the dorsolateral aspect of the STN. That region is near other regions of the brain, the stimulation of which, may cause side effects. For example, the dorsolateral aspect of the STN is near the internal capsule (IC), which contains the corticospinal tract. Stimulation that recruits neural elements in that region may cause side effects such as muscle contractions, speech problems (dysarthria), and the like.

Typically, once a lead is positioned in a patient and an effective location for providing therapy is determined, the clinician will titrate the stimulation amplitude upward (i.e., slowly increase the stimulation amplitude) until side effects are seen. The range of stimulation amplitudes between the lowest amplitude at which a benefit is seen and an amplitude at which intolerable side effects occur is referred to as a therapeutic window. A clinician would typically like to provide stimulation at a location that has a large therapeutic window. However, the algorithm 1100 of Figure 11 may not provide an indication of the therapeutic window because it does not necessarily reflect the presence of side effects. In other words, the presence and intensity of the recorded EPs may indicate that the stimulation is recruiting the target neural elements (for example in the STN) but does not indicate whether or not non-target neural elements are being recruited (for example, in the IC). Consequently, referring to Figure 12, location 1210₃, which provides the greatest EP response, may not be the location that provides the best therapeutic window because stimulation at that location may evoke side effects at relatively low stimulation amplitudes. There may be a better stimulation location on the lead that better balances good recruitment of target neural elements (and consequently good therapeutic efficacy) and little recruitment of non-target neural elements (and consequently fewer side effects).

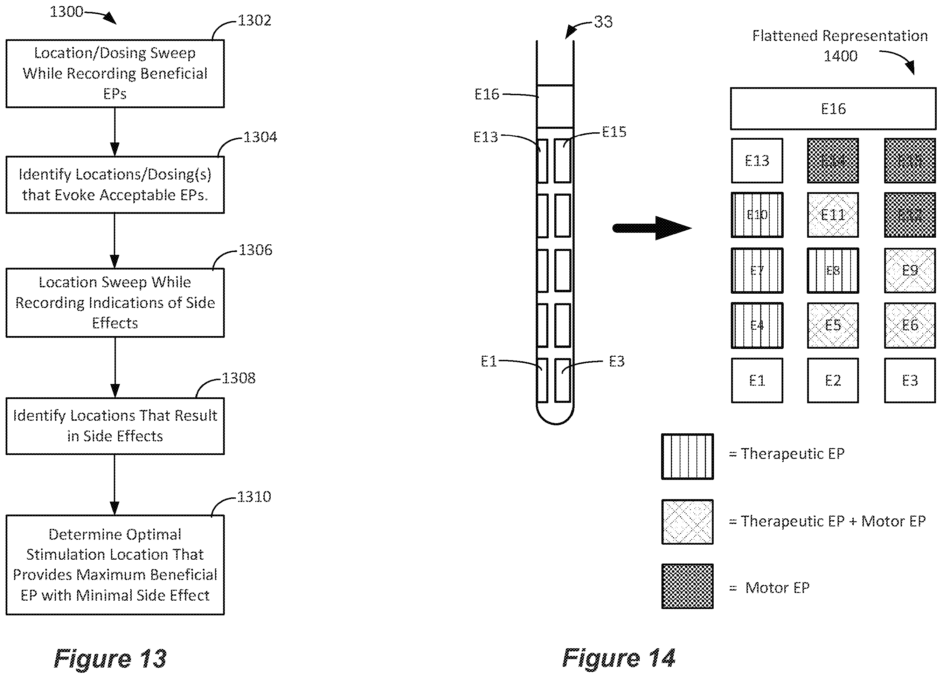

Figure 13 illustrates an improved algorithm 1300 for determining a stimulation location that seeks to balance recruitment of target neural elements while minimizing recruitment of non-target neural elements. At step 1302, locations on the lead are swept to determine stimulation locations/electrode configurations that result in therapeutic EPs. As mentioned above, the term "therapeutic EPs" refers to EPs that indicate stimulation that is likely to provide therapeutic benefit to the patient. According to some embodiments, performing step 1302 of the algorithm 1300 may be very similar to performing algorithm 1100 (Fig. 11). For example, the location sweep 1302 may comprises initializing default stimulation parameters and sweeping the parameters until therapeutic EPs are detectable. One or more of the stimulation parameters, such as amplitude, pulse width, and/or frequency may be adjusted and/or swept. Stimulation may then be applied using various locations along the lead, for example, sweeping along different longitudinal locations and then sweeping through different rotational locations. MICC and current fractionalization can be used to provide electrode configurations that result in stimulation locations that are between (i.e., not co-located with) physical electrodes. EPs evoked by the stimulation at each of the locations are recorded and stimulation locations that correspond to strong therapeutic EP signals are identified (step 1304). According to some embodiments, one or more EP features, as described above, may be extracted from the recorded EPs and used as a basis for comparing EP values evoked using different stimulation locations.

At step 1306 the sweep (longitudinal and rotational) are repeated. During this sweep the algorithm receives and records a signal indicative of side effects evoked at each of the stimulation locations. According to some embodiments, the side effects may be motor/movement related, for example if areas of the corticospinal tract are inadvertently stimulated. Indications of such side effects may be referred to herein as motor EPs (MEPs), meaning that they are evoke potentials (or other recorded signals) indicative of unintended motor activity. As used herein, the term motor EPs is contrasted to therapeutic EPs (TEPs), because TEPs are associated with beneficial or therapeutic stimulation, whereas MEPs are associated with side effects. According to some embodiments, such motor EPs may be recorded using electromyography (EMG), or the like, to measure motor activity in one or more locations in the patient's body. Alternatively (or additionally), muscle movement may be detected using one or more mechanical sensors (mechanomyography). According to some embodiments, other external sensors may be used, such as speech sensors to detect speech problems (dysarthria). According to some embodiments, motor EPs may be detected electrically near motor areas of the patient's brain, for example, using a cortical array placed in the primary motor cortex (M1) area of the brain that can detect motor activity correlated with specific side effects that manifests with movement of different body parts and that can be sensed from the signals recorded in the primary motor cortex on the pre-central gyrus anterior to the central sulcus of the brain (see figure added before the claims). There is a motor map very well established in literature that can guide the placement of the cortical array to the patient specific body location where the side effects manifest. Of note, 30 to 40% of the corticospinal projection originate in M1, and the corticobulbar projections as well. Neurons in the M1 modulate their firing rate several hundred milliseconds before the actual movement starts (see, e.g., Georgopoulos, et al, "On the relations between the direction of two-dimensional arm movements and cell discharge in primate motor cortex," J. Neurosci. 2, (1982) 1527-37). Using brains signals sensed from the M1 area, such as evoked potentials and local field potentials correlated with specific motor side effects episodes or activity recorded simultaneously with the dorsal STN ERNA can allow proper adjustment of the stimulation location and dosis (amplitude, pulse width and frequency) and determination of the optimal therapeutic window. Alternatively, the cortical array can be placed in the supplementary motor cortex (pre-motor cortex). According to some embodiments, motor EPs may be detected as electrical signals recorded in the patient's spinal column, i.e., an electrospinograph. According to some embodiments, motor EPs may be detected by sensing neurotransmitter levels in motor areas of the patient's brain, for example, using fast scan cyclic voltammetry (FSCV). According to some embodiments, specific MEP signals may be recorded when side effects take place. One or more detection algorithms may be configured to automatically indicate that the identified MEP is present (form example, in the M1 region) and then reduce the stimulation current to an amplitude such that the MEP disappears, but such that adequate TEPs are still present.

At step 1308, the signals indicative of side effects (i.e., the recorded motor EPs) are used to determine which stimulation locations evoke the strongest motor EPs and cause the most problematic side effects. At step 1310, the correlation between stimulation location (i.e., electrode configuration) and therapeutic EPs (from step 1304) and the correlation between stimulation location and motor EPs (from step 1308) are used to determine the optimal location on the lead to provide therapeutic stimulation. Various algorithms and/or multi-objective optimization functions may be used to determine the optimal location for providing stimulation based on balancing the stimulation location that yields the best TEP and that results in minimal, or at least acceptable MEP measurements. The details of the particular multi-objective optimization will depend on the particular implementation. For example, the algorithm may maximize the TEP parameters while minimizing the MEP (or other side effect signals). According to some embodiments the algorithm may involve keeping the side effect signals below a certain (acceptable) threshold and then searching the TEP space to find the maximum TEP value. According to some embodiments, a ratio of the TEP and MEP signals may be used and compared to a threshold, for example.

Figure 14 illustrates correlations of stimulation location with recorded therapeutic EPs and motor EPs. Figure 14 shows an electrode lead 33 comprising 15 split ring or directional electrodes (E1-E15) and a single ring electrode E16. Figure 14 also shows the configuration of electrodes in a flattened representation 1400. According to some embodiments, a representation of the electrodes, such as the flattened representation 1400 or the "heat map" representation illustrated in Figure 12 may be provided on a GUI of a computing device, such as the CP 70. The flattened representation 1400 shows correlations between the stimulating electrodes and the occurrence of therapeutic EPs and/or motor EPs. For example, when stimulation is applied at electrodes E1, E2, E3, E13 or E16 neither therapeutic EPs nor motor EPs are observed, or if they are observed they are not strong enough to consider. Thus, the representations of those electrodes are left blank. When stimulation is applied at electrodes E4, E7, E8, or E10, therapeutic EPs are recorded. When stimulation is applied at electrodes E12, E14, or E15 motor EPs are recorded. And when stimulation is applied at electrodes E5, E6, E9, or E11, both therapeutic and motor EPs are recorded. Thus, the representation 1400 might indicate that stimulation at one or more of electrodes E4, E7, E8, or E10 would be expected to provide the best therapy with the minimum of side effects.

Notice that the flattened representation 1400 only correlates therapeutic and motor EPs with discreet electrode locations. But as mentioned above, MICC/current steering can be used to provide electrode configurations that provide field shapes and stimulation locations that are located between the physical electrodes. According to some embodiments, the representation of the electrode array may be configured to correlate therapeutic and motor EPs with stimulation locations that do not coincide with physical electrode locations.

Also notice that the flattened representation 1400 is "binary" with respect to presence of therapeutic and motor EPs. In other words, the representation shows that indicates either the presence or absence of therapeutic and motor EPs. However, other embodiments of such representations may be configured with gradations, such as heat maps, color coding, and the like, configured to indicate the strength of the therapeutic EPs (and/or the values of features extracted from the therapeutic EPs) and/or the motor EPs. According to some embodiments, the representation may be configured to provide a visual indication of the stimulation amplitude at which motor EPs are first observed. Such a representation may provide an indication of the therapeutic window at that stimulation location, as explained in more detail below. In other words, being able to increase the stimulation to higher amplitudes without evoking side effects contributes to a greater therapeutic window.

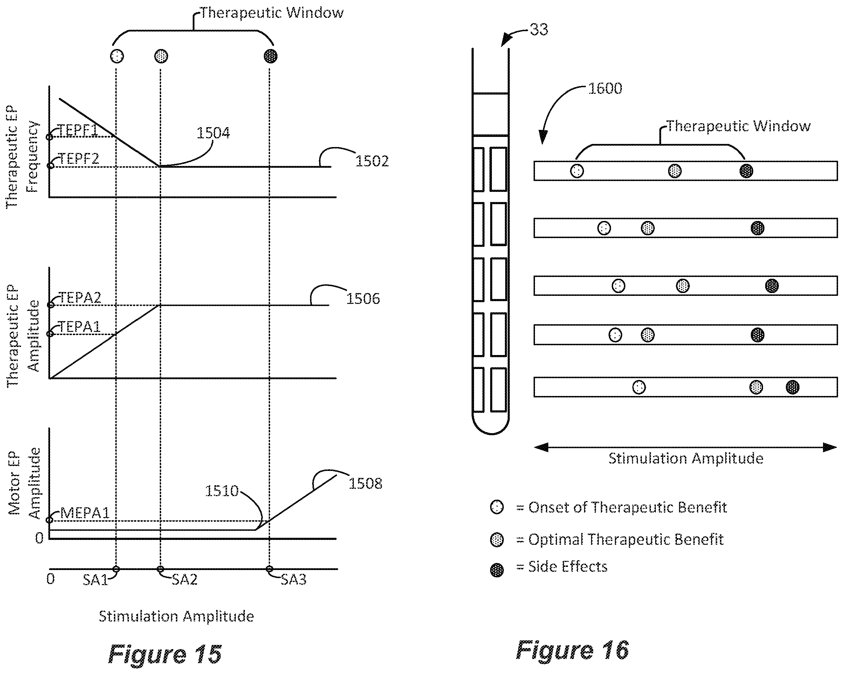

Figure 15 illustrates some aspects of how therapeutic EPs (TEPs) and motor EPs (MEPs) together can be used to indicate the therapeutic window for potential stimulation locations (i.e., electrode configurations). In Figure 15, assume that stimulation is being provided at a particular location on an implanted lead (i.e., using a particular electrode configuration). Figure 15 illustrates the stimulation amplitude dependence of two different features extracted from recorded TEPs evoked by the stimulation. In this example, the TEPs may be ERNAs, though they could be other EPs, as discussed above. The curve 1502 shows the behavior of the TEP frequency as a function of the stimulation amplitude. Assume that the clinician knows, either through modeling, experimentation, multi-patient studies, historical data, or the like, that a TEP frequency of TEPF1 is likely to correspond to the maximum frequency observed when the stimulation provides a therapeutic benefit. In other words, if the observed TEP frequency is greater than TEPF1, no therapeutic benefit is expected. The TEP frequency of TEPF1 corresponds to a stimulation amplitude SA1, which is the stimulation that provides the minimal therapeutic response.

In the illustrated example, the TEP frequency decreases as a function of stimulation amplitude until it reaches an inflection point (1504) and plateaus at a stimulation amplitude SA2. Assume that the TEP frequency of TEPF2 is expected to correspond to the optimal therapeutic response. Notice that increasing the stimulation amplitude beyond an amplitude of SA2 does not provide any better therapeutic response (i.e., it provides no further decrease in the TEP frequency). Thus, the stimulation amplitude of SA2 is taken to the optimum stimulation amplitude.

Curve 1506 of Figure 15 shows the behavior of the TEP amplitude as a function of the stimulation amplitude. Assume that TEP amplitude TEPA1 corresponds to the minimum TEP amplitude at which a therapeutic benefit is observed and TEPA2 corresponds to the TEP amplitude corresponding to the optimal therapeutic benefit. Notice that increasing the stimulation amplitude beyond SA2 provides no increase in the TEP amplitude and no additional therapeutic benefit.

Curve 1508 shows the behavior of one or more recorded MEPs as a function of the stimulation amplitude. Notice that the MEP amplitude is minimal until an inflection point 1510 is reached, after which the MEP amplitude increases as a function of the stimulation amplitude. Assume that the MEP amplitude MEPA1 is the maximum acceptable MEP amplitude. In other words, if the MEP amplitude exceeds MEPA1, then the side effects of the stimulation is not tolerable for the patient, for example. That MEP amplitude arises at a stimulation amplitude of SA3.

As shown in Figure 15, for a given stimulation location (i.e., a given electrode configuration) a clinician can use parameter values extracted from recorded TEPs, such as the values TEPF1 and/or TEPA1 to determine the stimulation amplitude that is the minimum amplitude for providing therapy. TEP parameter values, such as TEPF2 and TEPA2 may provide an indication of the stimulation amplitude corresponding to the optimal therapeutic stimulation. Values extracted from motor EPs (MEPs), such as MEPA1, can provide an indication of stimulation amplitudes that give rise to unacceptable side effects. In Figure 15, the amplitude range from SA1 (the minimum amplitude providing a therapeutic benefit) to SA3 (the maximum amplitude, beyond which side effects will occur) is the therapeutic window. The process illustrated in Figure 15 can be repeated for other stimulation locations (electrode configurations) to determine the therapeutic window for various potential stimulation locations. Notice that in Figure 15, the therapeutic window was determined with regard to stimulation amplitude. According to some embodiments, other stimulation parameters can be determined, for example, pulse width and/or stimulation frequency. Also, other TEP features besides amplitude and frequency can be used, as described above.

Figure 16 illustrates a display of therapeutic windows determined for stimulation applied at a number of different longitudinal locations on an electrode lead 33. Each of the segments 1600 may be determined as described with regard to Figure 15. While Figure 16 illustrates therapeutic window determinations for stimulation at various longitudinal rows of electrodes, it should be appreciated that the therapeutic window can be determined for stimulation at any location on the lead 33. For example, the therapeutic window can be determined for stimulation at different angular locations about the lead. Also, as mentioned above, current steering/MICC can provide stimulation at locations that do not coincide with physical electrodes. Therapeutic window can be determined for such stimulation locations. Embodiments of the disclosure provide methods and systems for displaying a representation of therapeutic windows as a function of various stimulation locations on an electrode lead, similar to the illustration of Figure 16. For example, such representations may be provided using a GUI of a computing device such as the CP 70.

Although particular embodiments of the present invention have been shown and described, it should be understood that the above discussion is not intended to limit the present invention to these embodiments. It will be obvious to those skilled in the art that various changes and modifications may be made without departing from the scope of the present invention.

## Claims

1. A system (70, 100) for providing stimulation to a patient's brain using an electrode lead (15, 33) that is implantable in the patient's brain and comprises a plurality of electrodes (16, E1-E16), the system comprising:
control circuitry configured to:
use one or more of the plurality of electrodes to sequentially provide electrical stimulation at different locations on the electrode lead,
for each stimulation location:
use one or more of the plurality of electrodes to record first signals, wherein the first signals are indicative of electric potentials evoked in the patient's brain by the stimulation, and
receive one or more second signals that are indicative of motor activity evoked by the stimulation, and
select an optimized location on the electrode lead for providing therapeutic electrical stimulation based on the first and second signals.

2. The system of claim 1, wherein the recorded first signals are indicative of evoked resonant neural responses evoked by the stimulation.

3. The system of claims 1 or 2, wherein the second signals are generated using electromyography, EMG, one or more mechanical sensors, speech sensors, and/or electrochemical sensors.

4. The system of any of claims 1-3, wherein the second signals are generated using a cortical array.

5. The system of any of claims 1-4, wherein the electric potentials evoked in the patient's brain are correlated with therapeutic efficacy of the stimulation.

6. The system of any of claims 1-5, wherein the motor activity evoked by the stimulation is correlated with an undesirable side effect of the stimulation.

7. The system of any of claims 1-6, wherein selecting an optimized location on the electrode lead for based on the first and second signals comprises using the first and second signals to determine a therapeutic window for each of the stimulation locations.

8. The system of any of claims 1-7, wherein the first signals are indicative of potentials evoked in the patient's subthalamic nucleus, STN.

9. The system of any of claims 1-8, wherein the second signals are indicative of recruitment of neural elements in the patient's corticospinal tract by the stimulation.

10. The system of any of claims 1-9, wherein selecting an optimized location on the electrode lead based on the first and second signals comprises:
for each stimulation location determining a value for a feature of the first signal and a value for a feature of the second signal,
selecting a plurality of stimulation locations where the value for the feature of the second signals is less than a threshold value, and selecting a stimulation location from the plurality of stimulation locations where the value for the feature of the first signal is the greatest.

11. The system of claim 10, wherein feature of the first signal is selected from on or more of a height of any peak, a peak-to-peak height between any two peaks, a ratio of peak heights, a peak width of any peak, and an area under any peak.

12. The system of any of claims 1-9, wherein selecting an optimized location on the electrode lead based on the first and second signals comprises:
for each stimulation location determining a ratio comprising a value for a feature of the first signal and a value for a feature of the second signal and comparing the ratio to a threshold value.

13. The system of claim 12, wherein feature of the first signal is selected from on or more of a height of any peak, a peak-to-peak height between any two peaks, a ratio of peak heights, a peak width of any peak, and an area under any peak.

14. The system of any of claims 1-13, wherein the control circuitry is further configured to adjust one or more parameters of the electrical simulation using the first and second signals.

15. The system of claim 14, wherein the one or more parameters are one or more of an electrode configuration, an amplitude, a pulse width, and a frequency.

## Patentansprüche

1. System (70, 100) zur Bereitstellung von Stimulation für das Gehirn eines Patienten mit Hilfe einer Elektrodenleitung (15, 33), die im Gehirn des Patienten implantierbar ist und mehrere Elektroden (16, E1-E16) aufweist, wobei das System aufweist:
eine Steuerschaltung, die so konfiguriert ist, dass sie:
eine oder mehrere der mehreren Elektroden verwendet, um elektrische Stimulation an unterschiedlichen Stellen auf der Elektrodenleitung nacheinander bereitzustellen,
für jede Stimulationsstelle:
eine oder mehrere der mehreren Elektroden verwendet, um erste Signale aufzuzeichnen,
wobei die ersten Signale elektrische Potenziale angeben, die durch die Stimulation im Gehirn des Patienten evoziert werden, und
ein oder mehrere zweite Signale empfängt, die motorische Aktivität angeben, die durch die Stimulation evoziert wird, und
eine optimierte Stelle auf der Elektrodenleitung zur Bereitstellung von therapeutischer elektrischer Stimulation auf der Grundlage der ersten und zweiten Signale auswählt.

2. System nach Anspruch 1, wobei die aufgezeichneten ersten Signale evozierte resonante Neuralreaktionen angeben, die durch die Stimulation evoziert werden.

3. System nach Anspruch 1 oder 2, wobei die zweiten Signale mit Hilfe von Elektromyographie, EMG, einem oder mehreren mechanischen Sensoren, Sprachsensoren und/oder elektrochemischen Sensoren erzeugt werden.

4. System nach einem der Ansprüche 1 bis 3, wobei die zweiten Signale mit Hilfe eines kortikalen Arrays erzeugt werden.

5. System nach einem der Ansprüche 1 bis 4, wobei die im Gehirn des Patienten evozierten elektrischen Potenziale mit der therapeutischen Wirksamkeit der Stimulation korreliert sind.

6. System nach einem der Ansprüche 1 bis 5, wobei die durch die Stimulation evozierte motorische Aktivität mit einer unerwünschten Nebenwirkung der Stimulation korreliert ist.

7. System nach einem der Ansprüche 1 bis 6, wobei das Auswählen einer optimierten Stelle auf der Elektrodenleitung auf der Grundlage der ersten und zweiten Signale aufweist: Verwenden der ersten und zweiten Signale, um ein therapeutisches Fenster für jede der Stimulationsstellen zu bestimmen.

8. System nach einem der Ansprüche 1 bis 7, wobei die ersten Signale Potenziale angeben, die im Nucleus subthalamicus, STN, des Patienten evoziert werden.

9. System nach einem der Ansprüche 1 bis 8, wobei die zweiten Signale die Rekrutierung von Neuralelementen im Tractus corticospinalis des Patienten durch die Stimulation angeben.

10. System nach einem der Ansprüche 1 bis 9, wobei das Auswählen einer optimierten Stelle auf der Elektrodenleitung auf der Grundlage der ersten und zweiten Signale aufweist:
für jede Stimulationsstelle erfolgendes Bestimmen eines Werts für ein Merkmal des ersten Signals und eines Werts für ein Merkmal des zweiten Signals,
Auswählen mehrerer Stimulationsstellen, an denen der Wert für das Merkmal der zweiten Signale kleiner ist als ein Schwellwert, und Auswählen einer Stimulationsstelle aus den mehreren Stimulationsstellen, an der der Wert für das Merkmal des ersten Signals am größten ist.

11. System nach Anspruch 10, wobei das Merkmal des ersten Signals ausgewählt wird aus einer Höhe eines Peaks, einer Peak-zu-Peak-Höhe zwischen zwei Peaks, einem Verhältnis von Peakhöhen, einer Peakbreite eines Peaks und/oder einer Fläche unter einem Peak.

12. System nach einem der Ansprüche 1 bis 9, wobei das Auswählen einer optimierten Stelle auf der Elektrodenleitung auf der Grundlage der ersten und zweiten Signale aufweist:
für jede Stimulationsstelle erfolgendes Bestimmen eines Verhältnisses mit einem Wert für ein Merkmal des ersten Signals und einem Wert für ein Merkmal des zweiten Signals und Vergleichen des Verhältnisses mit einem Schwellwert.

13. System nach Anspruch 12, wobei das Merkmal des ersten Signals ausgewählt wird aus einer Höhe eines Peaks, einer Peak-zu-Peak-Höhe zwischen zwei Peaks, einem Verhältnis von Peakhöhen, einer Peakbreite eines Peaks und/oder einer Fläche unter einem Peak.

14. System nach einem der Ansprüche 1 bis 13, wobei die Steuerschaltung ferner so konfiguriert ist, dass sie einen oder mehrere Parameter der elektrischen Stimulation mit Hilfe der ersten und zweiten Signale einstellt.

15. System nach Anspruch 14, wobei der eine oder die mehreren Parameter eine Elektrodenkonfiguration, eine Amplitude, eine Impulsbreite und/oder eine Frequenz sind.

## Revendications

1. Système (70, 100) pour fournir une stimulation au cerveau d'un patient à l'aide d'une sonde d'électrodes (15, 33) qui est implantable dans le cerveau du patient et comprend une pluralité d'électrodes (16, E1-E16), le système comprenant:
des circuits de commande configurés pour:
utiliser une ou plusieurs de la pluralité d'électrodes pour fournir séquentiellement une stimulation électrique à différents emplacements sur la sonde d'électrodes,
pour chaque emplacement de stimulation:
utiliser une ou plusieurs de la pluralité d'électrodes pour enregistrer des premiers signaux,
dans lequel les premiers signaux sont indicatifs de potentiels électriques suscités dans le cerveau du patient par la stimulation, et
recevoir un ou plusieurs deuxièmes signaux qui sont indicatifs d'une activité motrice suscitée par la stimulation, et
sélectionner un emplacement optimisé sur la sonde d'électrodes pour fournir une stimulation électrique thérapeutique sur la base des premiers et deuxièmes signaux.

2. Système selon la revendication 1, dans lequel les premiers signaux enregistrés sont indicatifs de réponses neuronales résonantes suscitées suscitées par la stimulation.

3. Système selon la revendication 1 ou 2, dans lequel les deuxièmes signaux sont générés au moyen d'une électromyographie, EMG, d'un ou plusieurs capteurs mécaniques, de capteurs de parole et/ou de capteurs électrochimiques.

4. Système selon l'une quelconque des revendications 1 à 3, dans lequel les deuxièmes signaux sont générés au moyen d'un réseau d'électrodes corticales.

5. Système selon l'une quelconque des revendications 1 à 4, dans lequel les potentiels électriques suscités dans le cerveau du patient sont corrélés à l'efficacité thérapeutique de la stimulation.

6. Système selon l'une quelconque des revendications 1 à 5, dans lequel l'activité motrice suscitée par la stimulation est corrélée à un effet secondaire indésirable de la stimulation.

7. Système selon l'une quelconque des revendications 1 à 6, dans lequel la sélection d'un emplacement optimisé sur la sonde d'électrodes sur la base des premier et deuxième signaux comprend l'utilisation des premier et deuxième signaux pour déterminer une fenêtre thérapeutique pour chacun des emplacements de stimulation.

8. Système selon l'une quelconque des revendications 1 à 7, dans lequel les premiers signaux sont indicatifs de potentiels suscités dans le noyau sous-thalamique, NST, du patient.

9. Système selon l'une quelconque des revendications 1 à 8, dans lequel les deuxièmes signaux sont indicatifs du recrutement d'éléments neuronaux dans le tractus corticospinal du patient par la stimulation.

10. Système selon l'une quelconque des revendications 1 à 9, dans lequel la sélection d'un emplacement optimisé sur la sonde d'électrodes sur la base des premier et deuxième signaux comprend:
pour chaque emplacement de stimulation, la détermination d'une valeur pour une caractéristique du premier signal et d'une valeur pour une caractéristique du deuxième signal,
la sélection d'une pluralité d'emplacements de stimulation où la valeur pour la caractéristique des deuxième signaux est inférieure à une valeur seuil, et la sélection d'un emplacement de stimulation parmi la pluralité d'emplacements de stimulation où la valeur pour la caractéristique du premier signal est la plus élevée.

11. Système selon la revendication 10, dans lequel la caractéristique du premier signal est choisie parmi une ou plusieurs d'une hauteur d'un pic quelconque, d'une hauteur pic à pic entre deux pics quelconques, d'un rapport de hauteurs de pics, d'une largeur de pic d'un pic quelconque et d'une aire sous un pic quelconque.

12. Système selon l'une quelconque des revendications 1 à 9, dans lequel la sélection d'un emplacement optimisé sur la sonde d'électrodes sur la base des premier et deuxième signaux comprend:
pour chaque emplacement de stimulation, la détermination d'un rapport comprenant une valeur pour une caractéristique du premier signal et une valeur pour une caractéristique du deuxième signal et la comparaison du rapport à une valeur seuil.

13. Système selon la revendication 12, dans lequel la caractéristique du premier signal est choisie parmi une ou plusieurs d'une hauteur d'un pic quelconque, d'une hauteur pic à pic entre deux pics quelconques, d'un rapport de hauteurs de pics, d'une largeur de pic d'un pic quelconque et d'une aire sous un pic quelconque.

14. Système selon l'une quelconque des revendications 1 à 13, dans lequel les circuits de commande sont configurés en outre pour ajuster un ou plusieurs paramètres de la simulation électrique à l'aide des premier et deuxième signaux.

15. Système selon la revendication 14, dans lequel les un ou plusieurs paramètres sont une ou plusieurs d'une configuration des électrodes, d'une amplitude, d'une largeur d'impulsion et d'une fréquence.
